# EUROPÄISCHE PATENTSCHRIFT

(11) **EP 1 560 608 B1**
(45) Veröffentlichungstag und Bekanntmachung des Hinweises auf die Patenterteilung: **16.06.2010**
(21) Anmeldenummer: 03775272.2
(22) Anmeldetag: 31.10.2003
(51) Int. Cl.: A61L 15/58, A61K 9/70, A61K 47/32

(54) **FEUCHTIGKEITSAKTIVIERBARE KLEBSTOFFE FÜR MEDIZINISCHE ANWENDUNGSZWECKE**
MOISTURE-ACTIVATED ADHESIVES FOR MEDICAL APPLICATIONS
ADHESIFS ACTIVABLES PAR HUMIDITE POUR USAGES MEDICAUX

(30) Priorität: 13.11.2002 DE 10252725
(43) Veröffentlichungstag der Anmeldung: 10.08.2005
(73) Patentinhaber: LTS LOHMANN Therapie-Systeme AG, 56626 Andernach (DE)
(72) Erfinder: RIEMENSCHNITTER, Marc, 79111 Freiburg i. Br. (DE)
(74) Vertreter: Flaccus, Rolf-Dieter
(86) Internationale Anmeldenummer: PCT/EP2003/012117
(87) Internationale Veröffentlichungsnummer: WO 2004/043504

(56) Entgegenhaltungen:
- WO-A-01/68045
- DE-A- 19 856 101
- US-A- 4 505 976
- US-B1- 6 375 963
- WOOLFSON A D ET AL: "Development and characterisation of a moisture-activated bioadhesive drug delivery system for percutaneous local anaesthesia" INTERNATIONAL JOURNAL OF PHARMACEUTICS 30 JUN 1998 NETHERLANDS, Bd. 169, Nr. 1, 30. Juni 1998 (1998-06-30), Seiten 83-94, XP002272600 ISSN: 0378-5173

## Beschreibung

Die vorliegende Erfindung betrifft feuchtigkeitsaktivierbare Klebstoffe für medizinische Anwendungszwecke, insbesondere für medizinische Pflaster und transdermalen therapeutische Systeme.
Die Erfindung umfaßt ferner medizinische Pflaster und transdermale therapeutische Systeme, die derartige feuchtigkeitsaktivierbare Klebstoffe enthalten.

Medizinische Pflaster und transdermale therapeutische Systeme (TTS) besitzen in der Regel eine Haftkleberschicht, durch welche eine selbstklebende Befestigung auf der Haut ermöglicht wird. Bei transdermalen therapeutischen Systemen hat die Haftkleberschicht häufig zugleich die Funktion eines Wirkstoffreservoirs, d. h. der Haftkleber enthält einen oder mehrere wirkstoffe, die während der Applikationsdauer an die Haut abgegeben werden.

Vielfach werden die haftklebenden Schichten der medizinischen Pflaster oder TTS aus Polymeren wie z. B. Polyacrylaten, Polyisobutylenen, Polyisoprenen oder dgl. hergestellt. Derartige Haftkleber haben allerdings den Nachteil, daß sie auf feuchtem Untergrund nur schlecht kleben. Deshalb kann es bei der Applikation des Pflasters oder TTS auf eine feuchte Hautstelle zu einer mangelhaften Verklebung zwischen der haftklebenden Matrixschicht des Pflasters oder TTS und der Haut des Patienten kommen. Dieses Problem besteht insbesondere bei stark schwitzenden Patienten oder bei Hautpartien, welche eine erhöhte Schweißabsonderung aufweisen. Durch die mangelhafte Verklebung kann sich das Pflaster oder TTS ganz oder teilweise ablösen, so daß die beabsichtigte Funktion nicht mehr erfüllt werden kann. Insbesondere kann dadurch bei TTS die Wirkstoffabgabe beeinträchtigt werden.

Wegen der haftklebenden Eigenschaften muß die zum Aufkleben auf die Haut bestimmte Schicht eines Pflasters oder TTS vor der Applikation mit einer ablösbaren Schutzfolie bedeckt werden. Ferner neigen herkömmlich, für Pflaster oder TTS verwendete Haftklebermassen teilweise stark zum "kalten Fluß", was während der Lagerung zu einem Austritt der Klebermasse aus dem Pflaster oder TTS und nachfolgend zu einem Festkleben an der Verpackung führen kann. Außerdem ist bei den herkömmlichen Haftklebermassen von Nachteil, daß sie fast ausschließlich mit organischen Lösemitteln angesetzt werden, was zu Problemen aufgrund des Rest-Lösemittelgehalts führen kann.

Weiterhin ist bei wirkstoffhaltigen Klebstoff-Matrixschichten zu beachten, daß eine übermäßig stark lipophile Umgebung sich zumindest bei einigen Wirkstoffen oder Wirkstoffgattungen negativ auf die Freisetzung der Wirkstoffe aus der Matrix auswirken kann. Dies hat zur Folge, daß die beabsichtigte Wirkstoff-Freisetzungsrate nicht mehr erreicht werden kann.

Der vorliegenden Erfindung lag deshalb die Aufgabe zugrunde, einen Klebstoff für medizinische Pflaster oder TTS bereitzustellen, der die vorstehend genannten Nachteile nicht aufweist und der insbesondere auf feuchtem Untergrund verbesserte Klebeigenschaften besitzt.

Diese Aufgabe wird überraschenderweise durch einen Klebstoff nach Anspruch 1 gelöst, sowie nach den in den Unteransprüchen beschriebenen bevorzugten Ausführungsformen.

Der erfindungsgemäße Klebstoff für medizinische Pflaster oder transdermale therapeutische Systeme enthält mindestens ein Polymethylvinylether-polymaleinsäureanhydrid-Copolymer in Kombination mit mindestens einem filmbildenden Polymer aus der Gruppe der nicht-haftklebenden Polyacrylate.

Von wesentlicher Bedeutung ist dabei, daß die Klebrigkeit der erfindungsgemäßen Klebstoffe durch Kontakt mit Feuchtigkeit oder durch Aufnahme von Feuchtigkeit aktiviert und/oder verstärkt wird. Dies bedeutet insbesondere, daß die aus einem erfindungsgemäßen Klebstoff hergestellte Klebstoffschicht (i) entweder zunächst keine selbstklebenden Eigenschaften hat und erst nach Anfeuchten oder durch Kontakt mit einer feuchten Unterlage (z. B. feuchte Hautstelle) klebrig wird, oder (ii) daß eine erfindungsgemäße Klebstoffschicht zwar an sich schon haftklebend ist, daß aber die Klebwirkung durch die Aufnahme von Feuchtigkeit verstärkt wird. Auf diese weise wird eine zuverlässige Klebwirkung auf feuchter oder schwitzender Haut gewährleistet. Der Begriff "feuchtigkeitsaktivierbar" bedeutet im Zusammenhang mit der vorliegenden Erfindung sowohl, daß die Klebrigkeit durch Feuchtigkeitseinwirkung initiiert werden kann, als auch, daß eine bestehende haftklebende Eigenschaft unter dem Einfluß von Feuchtigkeit verstärkt werden kann.

Durch das Prinzip der Feuchtigkeitsaktivierbarkeit ist es möglich, Klebschichten herzustellen, die im trockenen Zustand (z. B. während der Lagerung) nicht oder nur schwach adhäsiv sind, so daß auf eine Bedeckung dieser Klebschicht mit einer ablösbaren Schutzfolie verzichtet werden kann. Da die erfindungsgemäßen Klebschichten im trockenen Zustand nicht oder nur schwach adhäsiv sind, neigen sie auch nicht zu "kaltem Fluß", was ein weiterer vorteil ist, weil so das Ankleben der Pflaster, TTS etc. an der Verpackung verhindert wird.

Die Eigenschaft, daß die Klebwirkung durch Feuchtigkeitsaufnahme aktiviert oder verstärkt wird, ist im wesentlichen durch die erfindungsgemäße Rezeptur der Klebstoffzusammensetzung bedingt, d. h. durch einen Gehalt an bestimmten Komponenten oder Polymeren gemäß den Ansprüchen 1 bis 11. Zusätzlich ist dabei von Vorteil, daß hierbei überwiegend hydrophile Polymere zum Einsatz kommen, wie Polyvinylalkohole oder Cellulosederivate. Als weiterer Vorteil ergibt sich dadurch, daß die Herstellung der Klebermassen in vielen Fällen auf Wasserbasis oder mit wässrigen Lösungsmittelgemischen erfolgen kann, so daß auf die Verwendung organischer Lösungsmittel völlig oder weitgehend verzichtet werden kann. Dadurch können aufwendige Prüfungen zur Bestimmung des Rest-Lösemittelgehalts eingespart werden, es werden mögliche Haut-Reizwirkungen durch organische Lösemittel vermieden, und die Abluftentsorgungskosten bei der Herstellung werden vermindert.

Schließlich ist nach der Erfindung auch vorgesehen, den hydrophilen Charakter der Klebstoffe bzw. der daraus hergestellten Klebstoffschichten durch Zusatz weiterer hydrophiler Polymere oder hydrophiler Hilfsstoffe zu verstärken. Die erfindungsgemäßen Klebstoffe, bzw. die daraus hergestellten TTS, eignen sich deshalb besonders gut für die transdermale Verabreichung von Wirkstoffen, bei denen eine hydrophile Umgebung, d. h. ein hydrophiler Charakter der wirkstoffhaltigen Klebstoff-Matrix, das Freisetzungsverhalten des TTS in günstiger Weise beeinflußt.

Als optionale zusätzliche Bestandteile der erfindungsgemäßen, feuchtigkeitsaktivierbaren Klebstoff-Zusammensetzung eignen sich vorzugsweise Cellulosederivate, insbesondere Cellulosederivate aus der Gruppe, die Hydroxypropylmethylcellulose, Hydroxypropylcellulose, Natrium-Carboxymethylcellulose, Methylcellulose, Hydroxyethylcellulose und Hydroxypropylethylcellulose umfaßt.

Polymethylvinylether-polymaleinsäureanhydrid-Copolymere sind beispielsweise unter der Bezeichnung GANTREZ (Fa. ISP) erhältlich; bevorzugt werden Gantrez-AN-Typen (Anhydrid) verwendet. Daneben können mit Vorteil auch Salze oder die Säureformen der genannten Säureanhydride eingesetzt werden, wie z. B. Gantrez-S-Typen (Säureformen von AN-Typen, z. B. Gantrez S-97 BF;).

Die erfindungsgemäßen Klebstoffe enthalten nicht-haftklebende filmbildende Polyacrylate, wie z. B. Eudragit NE 40 D (Fa. Röhm); durch den Zusatz derartiger filmbildende, nicht-haftklebender Polymere kann eine Erhöhung der Klebedauer bewirkt werden und das Klebeverhalten günstig beeinflußt werden. Weitere geeignete filmbildende Polyacrylate sind dem Fachmann grundsätzlich bekannt.
Der Anteil des/der nicht haftklebenden Polymere, bezogen auf die Summe der Polymerbestandteile der Klebermasse, kann in einem weiten Bereich variiert werden, um die gewünschten Klebeigenschaften einzustellen; beispielsweise kann der Polyacrylat-Anteil in einer derartigen Formulierung 25-95 Gew.-%, vorzugsweise 50-80 Gew.-%, betragen.

Die Erfindung erfaßt des weiteren feuchtigkeitsaktivierbare Klebstoffzubereitungen, die zusätzlich einen Gehalt an einem Polyvinylalkohol aufweisen.

Weiterhin kann es sich zur Einstellung der gewünschten Klebeigenschaften als vorteilhaft erweisen, den erfindungsgemäßen feuchtigkeitsaktivierbaren Klebstoffen weitere polymere Bestandteile beizumischen, vorzugsweise Polymere aus der Polyvinylpyrrolidone, Gelatine, Stärke und Stärkederivate umfassenden Gruppe. Im übrigen eignet sich zur Herstellung der feuchtigkeitsaktivierbaren Klebstoffe grundsätzlich eine Vielzahl weiterer Bestandteile, sofern sie vergleichbare Eigenschaften aufweisen wie die hier genannten Polymere.

Die Erfindung sieht ferner vor, daß den Klebstoffzubereitungen bzw. den Klebschichten Hilfs- oder Zusatzstoffe beigefügt werden; hierbei kommen insbesondere Füllstoffe (z. B. SiO₂), Farbstoffe (z. B. TiO₂), Verdicker oder viskositätserhöhende Zusätze (z. B. Aerosil), Emulgatoren (z. B. polyethoxylierte Sorbitanfettsäureester wie TWEEN^{®} oder polyethoxylierte Fettalkohole wie BRIJ^{®}), Weichmacher (z. B. Polyethylenglykol, Glycerin), Süßstoffe (z. B. Sorbitol, Aspartam, Saccharin), Aromastoffe, Konservierungsmittel (z. B. Sorbinsäure und deren Salze) und Trockenmittel (z. B. Natriumsulfat) in Betracht.

Als weitere Zusatzstoffe werden bevorzugt organische Säuren, insbesondere aus den Gruppen der gesättigten Alkanmonocarbonsäuren, der gesättigten Alkandicarbonsäuren und der Hydroxyalkansäuren (z. B. Weinsäure) verwendet. Die Anzahl der C-Atome liegt bei diesen Carbonsäuren vorzugsweise im Bereich von 2 bis 20. Bei Zusatz von organischen Säuren, bevorzugt Carbonsäuren, kommt es durch Wechselwirkungen zwischen Säure und Cellulosederivaten, insbesondere mit Natrium-Carboxymethylcellulose, z. B. durch teilweise Veresterung, zu einer Erhöhung der Naßklebkraft sowie zu einer Verlägerung der Klebedauer.

Der gesamte Anteil an Polymeren beträgt bei den erfindungsgemäßen Klebstoffen oder den daraus hergestellten Klebstoffschichten vorzugsweise 45-99 Gew.-%, bezogen auf die Klebstoffmasse oder die Klebstoffschicht; der Rest besteht aus Hilfs- oder Zusatzstoff (en) und/oder Wirkstoff(en), sowie gegebenenfalls einem Lösemittel-Anteil. Als Lösemittel für die erfindungsgemäßen Klebstoffzubereitungen kommen bevorzugt Wasser, wässerige Lösemittelgemische, Alkohole, Ester (wie z. B. Ethylacetat) und andere polare Lösemittel in Betracht,

Besonders vorteilhaft sind außerdem diejenigen Klebstofformulierungen, welche zusätzlich zu der erfindungsgemäßen feuchtigkeitsaktivierbaren Kleberformulierung ein oder mehrere haftklebende Polymere enthalten. Dadurch weisen derartige Klebstoffe und die daraus erhaltenen Klebschichten sowohl die Vorteile herkömmlicher Haftkleber auf, als auch diejenigen Vorteile, die durch die erfindungsgemäßen feuchtigkeitsaktivierbaren Kleberzusammensetzungen bedingt sind. Die erfindungsgemäßen Klebstoffe, welche zusätzlich haftklebende Polymere enthalten, zeichnen sich insbesondere dadurch aus, daß ihre Klebkraft unter Feuchtigkeitseinfluß aktiviert und/oder sogar verstärkt wird. Gleichzeitig verfügen sie auch in Abwesenheit von Hautfeuchtigkeit über ein ausreichendes Klebvermögen.

Als haftklebende Polymere werden vorzugsweise solche aus der Gruppe der Polyacrylate, Polyisobutylen und Polyisoprene, Silikonkleber und Schmelz-Haftkleber umfassenden Gruppe ausgewählt. Als Polyacrylate werden Polymere auf der Basis von Acrylsäure bzw. Methacrylsäure und deren Estern verstanden, sowie Gemische solcher Polymere. Geeignete haftklebende Polyacrylate sind dem Fachmann bekannt (z. B. "Duro-Tak 326-2353"; Fa. National Starch & Chemical B.V.).

Die erfindungsgemäßen feuchtigkeitsaktivierbaren Klebstoff-Formulierungen lassen sich mit vorteil zur Herstellung medizinischer Pflaster oder transdermaler therapeutischer Systeme (TTS) verwenden. Derartige Pflaster und Systeme weisen mindestens eine feuchtigkeits-aktivierbare Klebstoff-Matrixschicht auf, welche aus einem erfindungsgemäßen Klebstoff hergestellt ist oder einen solchen enthält; ebenso lassen sich auch Mischungen solcher Klebstoffe verwenden. Der Aufbau solcher Pflaster oder TTS ist dem Fachmann grundsätzlich bekannt; er umfaßt neben der/den genannten Klebschicht(en) eine Träger- oder Rückschicht (z. B. eine Kunststoffolie wie PET-Folie oder textiles Material), auf welche die Klebstoffschicht aufgetragen ist. Die auf der Haut klebende Seite der Klebstoffschicht ist vor der Applikation üblicherweise mit einer abhäsiv beschichteten schutzfolie bedeckt; allerdings kann auf diese auch verzichtet werden, wie oben erwähnt.

Grundsätzlich lassen sich die erfindungsgemäßen feuchtigkeitsaktivierbaren Systeme in all denjenigen Fällen einsetzen, in denen ein flächenförmiger Gegenstand für eine bestimmte Zeitdauer auf einer Unterlage, insbesondere auf einer feuchten Unterlage, befestigt werden muß.

Im Falle eines TTS enthält die aus einer (oder mehreren) erfindungsgemäßen feuchtigkeitsaktivierbaren Haftkleberformulierung(en) hergestellte Matrixschicht mindestens einen Wirkstoff. Dieser kann in der Matrixschicht gelöst, dispergiert, als Emulsion oder in fester Form vorliegen. Als Wirkstoffe werden grundsätzlich alle Stoffe, Stoffgemische oder Zubereitungen verstanden, die für eine topische oder transdermale Verabreichung geeignet sind, und die im menschlichen oder tierischen Organismus eine physiologische Wirkung hervorrufen können, insbesondere Arzneimittel-Wirkstoffe, Hormone, Spurenelemente, Enzyme und Antigene. Die Wirkstoffe können der therapeutischen, prophylaktischen oder kosmetischen Behandlung dienen.

Insbesondere kommen folgende Wirkstoffe bzw. wirkstoffgruppen in Betracht:
1. Salze basischer oder saurer Wirkstoffe aus der Gruppe der ACE-Hemmstoffe, Anabolika, Antidiabetika, Antihypertonika, Antiinfektiva, Antikoagulantien, Antirheumatika, Diuretika, Hormone, Immunsupressiva, Laxantien, Lipidsenker, ZNS-aktive Verbindungen, Anti-Epileptika, Antihypertonika, Koronartherapeutika, etc.;
2. Substanzen, deren Aktivität durch den zutritt von wasser erhöht wird, wodurch höhere Wirkstoff-Fluxraten entstehen;
3. Hydrophile Wirkstoffe, die in hydrophoben Polymeren schlecht löslich sind, z. B. Insulin, Erythropoietin, Wachstumsfaktoren, Gonadoliberine, Oxytocin, Prolactin, Calcitonin, Parathyrin (Parathormon), Somatomadin, Melanotropin.

Gemäß einer weiteren bevorzugten Ausführungsform ist vorgesehen, daß die feuchtigkeitsaktivierbare Klebstoff-Matrix eines medizinischen Pflasters, insbesondere aber eines TTS, mit einem Überpflaster verbunden ist. Dieses weist eine Flächenausdehaung auf, welche diejenige der feuchtigkeitsaktivierbaren Klebstoff-Matrix übersteigt; vorzugsweise überragt das Überpflaster die Fläche der Matrixschicht nach allen Seiten hin. Des weiteren ist es vorteilhaft, auch das Überpflaster auf der hautzugewandten Seite mit einer haftklebenden Polymerschicht auszustatten.

Die Erfindung wird nachfolgend anhand eines Formulierungsbeispiels veranschaulicht:

### Beispiel :

Feuchtigkeitsaktivierbarer Klebstoff mit filmbildendem Polyacrylat

| Bestandteil | Mengen-Anteil |
|---|---|
| Eudragit NE 40 D | 71,0 Gew.-% |
| Gantrez AN-169 | 28,6 Gew.-% |
| Sorbitol | 0,4 Gew.-% |
| Ethanol | |

| | |
|---|---|
| Eudragit NE 40 D: nicht haftklebendes, filmbildendes Polyacrylat (Fa. Röhm) Gantrez AN-169: s. Beispiel 1. | |

### Herstellung:

Ethanol wird in einem geeigneten Ansatzgefäß vorgelegt und Sorbitol unter Rühren und Homogenisieren eingestreut. Eudragit NE 40 D sowie Gantrez AN-169 werden unter Rühren eingebracht der Ansatz wird bei ca. 50-70 °C gerührt, bis eine homogene Masse entsteht.

Die nach dem obigen Beispiel hergestellte Masse wird mit einer Erichsen-Rakel, einem Streichkasten oder mit einem Auftragswerk auf eine geeignete Prozeßfolie beschichtet und anschließend im Trokkenschrank oder im Trockenkanal getrocknet.

Der nach dem obigen Beispiel hergestellte Klebstoff-Film ist nicht selbstklebend, sondern die Klebrigkeit wird erst durch Befeuchtung der vorgesehenen Klebestelle oder nach Anfeuchten der Polymermatrix sowie bei Kontakt mit einem feuchten Substrat aktiviert.

In Vorversuchen wurden mit der obigen Beispielformulierung Klebezeiten im Bereich von 12-24 h auf der Haut erreicht.
Nach dem Ablösen der Wirkstoffpflaster, welche einen Klebstoff-Film nach den obigen Beispielen enthielten, von der Haut waren keine Rückstände auf der Haut vorhanden, oder die Rückstände ließen sich leicht mit Wasser abwaschen.

Durch die in den Unteransprüchen beschriebenen Modifikationen läßt sich die Klebwirkung weiter optimieren.

## Patentansprüche

1. Klebstoff für medizinische Pflaster oder transdermale therapeutische Systeme, wobei der Klebstoff eine Klebrigkeit aufweist, die durch Kontakt mit Feuchtigkeit oder durch Aufnahme von Feuchtigkeit aktiviert und/oder verstärkt wird, und **dadurch gekennzeichnet ist, daß** er mindestens ein Polymethylvinylether-polymaleinsäureanhydrid-Copolymer in Kombination mit mindestens einem filmbildende Polymer aus der Gruppe der nichthaftklebenden Polyacrylate enthält.

2. Klebstoff nach Anspruch 1, **dadurch gekennzeichnet, daß** er einen oder mehrere Polyvinylalkohol(e) enthält.

3. Klebstoff nach Anspruch 1 oder 2, **dadurch gekennzeichnet, daß** er mindestens ein Cellulosederivat enthält, vorzugsweise ausgewählt aus der Hydroxypropylmethylcellulose, Hydroxypropylcellulose, Natrium-Carboxymethylcellulose, Methylcellulose, Hydroxyethylcellulose und Hydroxypropylethylcellulose umfassenden Gruppe.

4. Klebstoff nach einem der Ansprüche 1 bis 3, **dadurch gekennzeichnet, daß** er mindestens einen Bestandteil aus der Polyvinylpyrrolidone, Gelatine, Stärke und Stärkederivate umfassenden Gruppe enthält.

5. Klebstoff nach einem oder mehreren der vorangehenden Ansprüche, **dadurch gekennzeichnet, daß** er zusätzlich ein oder mehrere haftklebende Polymere enthält, welche vorzugsweise aus der Polyacrylate, Polyisobutylene, Polyisoprene und Silikonkleber umfassenden Gruppe ausgewählt sind.

6. Klebstoff nach Anspruch einem der vorangehenden Ansprüche, **dadurch gekennzeichnet, daß** der Polyacrylat-Anteil 25-95 Gew.-%, vorzugsweise 50-80 Gew.-% beträgt, bezogen auf die Summe der in Anspruch 1 genannten Polymerkomponenten.

7. Klebstoff nach einem oder mehreren der vorangehenden Ansprüche, **dadurch gekennzeichnet, daß** er eine oder mehrere organische Säuren enthält, vorzugsweise aus der Gruppe, welche gesättigte Alkanmonocarbonsäuren, gesättigte Alkandicarbonsäuren und Hydroxyalkansäuren umfaßt.

8. Medizinisches Pflaster oder transdermales therapeutisches System, **dadurch gekennzeichnet, daß** es mindestens eine feuchtigkeits-aktivierbare Klebstoff-Matrixschicht aufweist, die einen Klebstoff nach einem der Ansprüche 1 bis 7 enthält.

9. Transdermales therapeutisches System nach Anspruch 8, **dadurch gekennzeichnet, daß** die genannte Matrixschicht mindestens einen Wirkstoff enthält.

10. Transdermales therapeutisches System nach Anspruch 9, **dadurch gekennzeichnet, daß** die genannte wirkstoffhaltige Matrixschicht mit einem Überpflaster verbunden ist, wobei das Überpflaster vorzugsweise mit einer haftklebenden Polymerschicht ausgestattet ist.

11. Verwendung eines Klebstoffes nach einem der Ansprüche 1 bis 10 zur Herstellung eines medizinischen Pflasters oder eines transdermalen therapeutischen Systems.

## Claims

1. Adhesive for medical patches or for transdermal therapeutic systems, said adhesive having a tackiness which is activated and/or increased by contact with moisture or by absorption of moisture, and said adhesive being **characterized in that** it contains at least one polymethyl vinyl ether-polymaleic acid anhydride copolymer in combination with at least one film-forming polymer from the group of the non-pressure-sensitive adhesive polyacrylates.

2. Adhesive according to claim 1, **characterised in that** it contains one or more polyvinyl alcohol(s).

3. Adhesive according to claim 1 or 2, **characterised in that** it contains at least one cellulose derivative, preferably selected from the group which comprises hydroxypropyl methyl cellulose, hydroxypropyl cellulose, sodium carboxymethyl cellulose, methyl cellulose, hydroxyethyl cellulose and hydroxypropyl ethyl cellulose.

4. Adhesive according to any one of claims 1 to 3, **characterised in that** it contains at least one component from the group comprising polyvinyl pyrrolidones, gelatine, starch and starch derivatives.

5. Adhesive according to one or more of the preceding claims, **characterised in that** it additionally contains one or more pressure-sensitive adhesive polymers preferably selected from the group comprising polyacrylates, polyisobutylenes, polyisoprenes and silicone adhesives.

6. Adhesive according to any one of the preceding claims, **characterised in that** the polyacrylate portion is 25-95%-wt, preferably 50-80%-wt., relative to the sum of the polymer components mentioned in claim 1.

7. Adhesive according to one or more of the preceding claims, **characterised in that** it contains one or more organic acids, preferably selected from the group comprising saturated alkanoic monocarboxylic acids, saturated alkanoic dicarboxylic acids and hydroxyalkanoic acids.

8. Medical patch or transdermal therapeutic system, **characterised in that** it is provided with at least one moisture-activatable adhesive matrix layer which contains an adhesive according to any one of claims 1 to 7.

9. Transdermal therapeutic system according to claim 8, **characterised in that** the said matrix layer contains at least one active substance.

10. Transdermal therapeutic system according to claim 9, **characterised in that** the said active substance-containing matrix layer is connected with an overlying patch, the overlying patch preferably being provided with a pressure-sensitive adhesive polymer layer.

11. The use of an adhesive according to any one of claims 1 to 10 for the manufacture of a medical patch or of a transdermal therapeutic system.

## Revendications

1. Adhésif pour des emplâtres médicaux ou pour des systèmes thérapeutiques transdermiques, l'adhésif présentant un caractère collant qui est activé et/ou renforcé par contact avec de l'humidité ou par absorption de l'humidité, et **caractérisé en ce qu'**il contient au moins un copolymère d'éther polyméthylvinylique-anhydride de l'acide polymaléique en combinaison avec au moins un polymère filmogène du groupe des polyacrylates non auto-adhésifs.

2. Adhésif selon la revendication 1, **caractérisé en ce qu'**il contient un ou plusieurs alcools polyvinyliques.

3. Adhésif selon la revendication 1 ou 2, **caractérisé en ce qu'**il contient au moins un dérivé de la cellulose, de préférence choisi parmi le groupe comprenant l'hydroxypropylméthylcellulose, l'hydroxypropylcellulose, la carboxyméthylcellulose de sodium, la méthylcellulose, l'hydroxyéthylcellulose et l'hydroxypropyléthylcellulose.

4. Adhésif selon l'une quelconque des revendications 1 à 3, **caractérisé en ce qu'**il contient au moins un constituant du groupe comprenant des polyvinylpyrrolidones, la gélatine, l'amidon et des dérivés de l'amidon.

5. Adhésif selon une ou plusieurs des revendications précédentes, **caractérisé en ce qu'**il contient en outre un ou plusieurs polymères auto-adhésifs, qui sont choisis de préférence parmi le groupe comprenant des polyacrylates, des polyisobutylènes, des polyisoprènes et des adhésifs à base de silicone.

6. Adhésif selon l'une quelconque des revendications précédentes, **caractérisé en ce que** la fraction de polyacrylate s'élève de 25 à 95 % en poids, de préférence de 50 à 80 % en poids, rapportés à la somme des composants polymères mentionnés à la revendication 1.

7. Adhésif selon une ou plusieurs des revendications précédentes, **caractérisé en ce qu'**il contient un ou plusieurs acides organiques, de préférence parmi le groupe qui comprend des acides alcane-monocarboxyliques saturés, des acides alcane-dicarboxyliques saturés et des acides hydroxyalcanoïques.

8. Emplâtre médical ou système thérapeutique transdermique, **caractérisé en ce qu'**il contient au moins une couche matricielle d'adhésif activable en présence d'humidité, qui contient un adhésif selon l'une quelconque des revendications 1 à 7.

9. Système thérapeutique transdermique selon la revendication 8, **caractérisé en ce que** la couche matricielle mentionnée contient au moins une substance active.

10. Système thérapeutique transdermique selon la revendication 9, **caractérisé en ce que** la couche matricielle mentionnée contenant une substance active est reliée à un emplâtre de recouvrement, l'emplâtre de recouvrement étant de préférence muni d'une couche polymère auto-adhésive.

11. Utilisation d'un adhésif selon l'une quelconque des revendications 1 à 10 pour la fabrication d'un emplâtre médical ou d'un système thérapeutique transdermique.
